# EUROPEAN PATENT SPECIFICATION

(11) **EP 3 731 824 B1**
(45) Date of publication and mention of the grant of the patent: **15.03.2023**
(21) Application number: 18899013.9
(22) Date of filing: 27.12.2018
(51) Int. Cl.: A61K 9/20, A61K 31/4439

(54) **PHARMACEUTICAL TABLET COMPOSITIONS OF DABIGATRAN**
MEHRSCHICHTIGE TABLETTENZUSAMMENSETZUNGEN VON DABIGATRAN
COMPOSITIONS PHARMACEUTIQUES DE DABIGATRAN SOUS FORME DE COMPRIMÉS

(30) Priority: 28.12.2017 TR 201722630
(43) Date of publication of application: 04.11.2020
(73) Proprietor: Sanovel Ilac Sanayi Ve Ticaret Anonim Sirketi, 34460 Sariyer/Istanbul (TR)
(72) Inventor: TÜRKYILMAZ, Ali, 34460 Istanbul (TR); PALANTÖKEN, Arzu, 34398 Istanbul (TR); GÜLKOK, Yildiz, 34460 Istanbul (TR); YAVUZ, Büsra, 34460 Istanbul (TR)
(74) Representative: Sevinç, Erkan
(86) International application number: PCT/TR2018/050919
(87) International publication number: WO 2019/151966

(56) References cited:
- EP-A1- 1 870 100
- EP-A1- 3 332 771
- EP-B1- 1 870 100
- WO-A1-2015/145462
- CN-A- 105 919 962

## Description

### Technical Field

The present invention relates to pharmaceutical tablet compositions for oral administration comprising dabigatran etexilate free base or pharmaceutically acceptable salts of dabigatran etexilate and at least one pharmaceutically acceptable excipient.

### Background of the Invention

Dabigatran etexilate (Formula 1), which is already known from the patent applicaton numbered WO 98/37075, is a direct thrombin inhibitor indicated to reduce the risk of stroke and systemic embolism in patients with non-valvular atrial fibrilation. Thrombin is a multifunctional enzyme which converts fibrinogen to fibrin, cross-linking fibrin monomers via activation of factor XIII and augmenting further thrombin production via the activation of factors V and VIII. It also activates platelets, generates anticoagulant activity via activation of protein C and initiates numerous cellular processes.

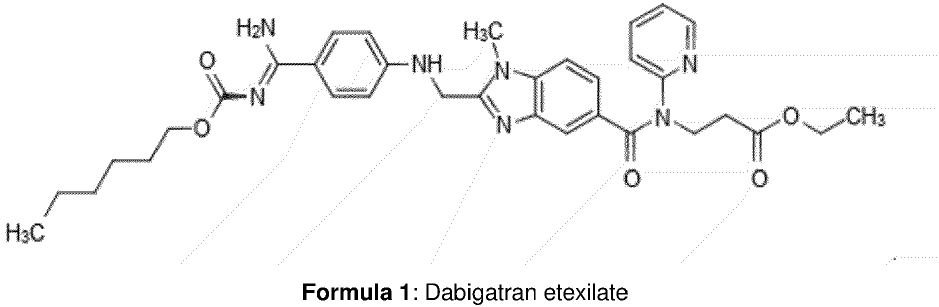

The methane sulphonic acid addition salt of dabigatran etexilate, which is commercially available under the trade name PRADAXA^{®} immediate release capsule (in the strength of 75, 110, 150 mg), is disclosed in the patent document EP1870100B1, wherein also disclosed, pellet formulation of dabigatran etexilate methanesulphonate. This composition is formulated with a core material consisting of organic acid and an active layer which encloses the core. Each PRADAXA^{®} capsule contains the following inactive ingredients: acacia, dimethicone, hypromellose, hydroxypropylcellulose, tartaric acid, carrageenan, potassium chloride, talc, titanium dioxide, and gelatin.

Apart from the methanesulfonate salt of dabigatran etexilate, other acid addition salts of the compound are provided in prior art. For example, WO2012/077136 is directed to the oxalate salt of dabigatran etexilate and besides, its hydrochloride salt is identified in EP1877395.

WO 2015/145462 A1 discloses compressed components of organic acid on one hand and dabigatran etexilate on the other hand, within one capsule.

It is known that the solubility of weakly basic drugs, such as dabigatran and dabigatran etexilate, is pH-dependent and may be increased by the provision of an acidic environment. Therefore, weakly basic drugs are formulated with an acidic excipient.

Dabigatran etexilate is also less stable in acidic environment. To avoid this stability problem, many solutions were offered in the prior art. Separating layer between the acidic core and the active substance layer is the most preferred way. In this invention, tablet-in-tablet dosage form is used and the core tablet comprising acidic excipient is coated.

In the prior art, most of formulations are in the form of capsules. But it is known that capsules cost more than tablets and have significant space and potency limitations since their powdered contents cannot be compressed to a significant degree. Since capsules are not airtight, their shelf-life is shorter than tablets.

Patent application EP1658056 discloses a conventional tablet formulation comprising dabigatran etexilate, organic acid with a solubility in water of > 1 g / 250 ml at 20°C together with conventional excipients and fillers. But in the patent application EP1658056, there is no insulating layer between the active agent and the organic acid.

WO03/074056 discloses a composition containing the following components: a core material comprising an organic acid, an insulating layer which separates the acid core from the layer containing the active substance and an active agent layer.

Patent application WO2012001156 relates to a process for the preparation of a solid oral dosage form comprising dabigatran etexilate. In this application, the pellets comprise a neutral core comprised of sucrose, microcrystalline cellulose or starch, or a commercially available tartaric acid pellet; the tartaric acid layer, the isolating layer, the active pharmaceutical ingredient layer and, optionally, an overcoat.

Patent application WO2013124340 discloses dabigatran etexilate compositions comprising a mixture of at least two types of particles and optionally at least one pharmaceutically acceptable excipient, wherein the first type of particles comprises the active agent; the second type of particles comprise at least one pharmaceutically acceptable organic acid; and optionally at least one type of particles are coated with a protective layer. In this patent application, the invention provides a quick dissolution particularly at earlier time points as compared to formulation having one type of particles/pellets.

The pellet formulation used in the marketed product Pradaxa^{®} is produced by a difficult production method which has a lot of production steps causing the process being longer than others. In this present invention, the pharmaceutical compositions have easy processes. Thus, there is still a need in the art to develop stable and bioavailable pharmaceutical formulations comprising dabigatran etexilate free base or pharmaceutically acceptable salts of dabigatran etexilate with a long shelf-life and a pH independent release by simplified and cost-effective processes. By this need, the formulation has been developed to overcome the solubility and stability problems of dabigatran etexilate in a safe manner disclosed above.

### Detailed Description of the Invention

The main object of the present invention is to provide pharmaceutical tablet compositions for oral administration comprising dabigatran etexilate free base or a pharmaceutically acceptable salt of dabigatran etexilate and at least one pharmaceutically acceptable excipient.

Multi-layered tablets are known as novel drug delivery systems. Compaction of different granules in the form of various layers in single tablets are called as multi-layered tablets. It generally consists of parallel, clear, coloured, visual distinct layers two to three or more APIs or APIs along with functional or non-functional placebo layers. Multi-layered tablet dosage forms are designed for a variety of reasons such as incompatibility problems between active agents or excipients.

In this present invention, the pharmaceutical compositions comprise:
a. a core tablet comprising an organic acid and at least one pharmaceutically acceptable excipient
b. a compressed outer tablet layer covering the core tablet, comprising dabigatran etexilate free base or a pharmaceutically acceptable salt of dabigatran etexilate and at least one pharmaceutically acceptable excipient
wherein the core tablet has a coating and the weight ratio of the core tablet to the compressed outer tablet layer is between 0.1 and 0.5.

In one embodiment, the pharmaceutical composition is in the form of a tablet-in-tablet.

It has been observed that, the dissolution and stability problems of the active agent are overcome by choosing tablet-in-tablet dosage form. Dissolution problem of the active agent is solved by using tablet-in-tablet dosage form which enables the pH-independent active agent to be formulated with an acidic excipient. Furthermore, there is a tablet coating between the core tablet comprising organic acid and the compressed outer tablet layer comprising the active agent to avoid incompatibility problems between them.

As used herein, the term "dabigatran etexilate free base" refers to dabigatran etexilate which is free from other forms of the active moiety, especially acid addition salts.

As used herein, the term "compressed outer tablet layer" means that the outer tablet layer of the tablet-in-tablet composition is formed by compression of a solid mixture, such as a direct blend, dry granulation, or wet granulation, rather than forming the outer layer by coating with a suspension or solution.

According to one embodiment, dabigatran etexilate free base or a pharmaceutically acceptable salt of dabigatran etexilate is present in the outer tablet layer in an amount of between 5.00 and 60.00 %, preferably between 10.00 and 50.00 % and more preferably this amount is 15.00 to 40.00 % by weight of the compressed outer tablet layer.

In the current invention, the weight ratio of the core tablet to the compressed outer tablet layer is between 0.1 and 0.5.

When the weight ratio of the core tablet to the compressed outer tablet layer comprising dabigatran etexilate free base or a pharmaceutically acceptable salt thereof is between 0.1 and 0.5, the stability of the composition increases.

Suitable coating materials are selected from the group comprising hydroxypropylmethyl cellulose (hypromellose), hydroxypropyl cellulose, polyvinyl alcohol (PVA), polyethylene glycol (PEG), talc, polyvinyl alcohol-polyethylene glycol copolymers (Kollicoat IR), ethylcellulose dispersions (Surelease), polyvinylprolidone, polyvinylprolidone-vinyl acetate copolymer (PVP-VA) and all kinds of OpadryTM, pigments, dyes, titanium dioxide, iron oxide or polymethylmetacrylate copolymers (Eudragit)or mixtures thereof.

In one preferred embodiment, the coating material is selected from hydroxypropylmethyl cellulose (hypromellose), polyethylene glycol (PEG) or mixtures thereof.

In one preferred embodiment, the coating material is Opadry Clear YS-1R-7006.

Selection of Opadry Clear YS-1R-7006 as the tablet coating between the core tablet and the compressed outer tablet layer helps to have an improved stability by preventing any contact between the organic acid and the active agent.

According to another embodiment, dabigatran etexilate free base or pharmaceutically acceptable salts of dabigatran etexilate is present in the dosage form in an amount of between 30 to 350 mg, preferably 50 to 300 mg and more preferably 50 to 250 mg.

According to one embodiment, at least one pharmaceutically acceptable excipient is selected from a group comprising disintegrants, binders, fillers, lubricants, glidants or mixtures thereof.

Suitable disintegrants are selected from a group comprising pregelatinized starch, cross-linked carboxymethyl cellulose (croscarmellose sodium), low-substituted hydroxypropyl cellulose (L-HPC), microcrystalline cellulose, sodium starch glycolate, starch, crospovidone (cross-linked polyvinyl N-pyrrolidone), poloxamer, sodium carboxymethyl cellulose, calcium carboxymethyl cellulose, carboxymethyl cellulose, docusate sodium, guar gum, polyacryline potassium, sodium alginate, alginic acid, alginates, ion-exchange resins, magnesium aluminium silica, sodium glycine carbonate, sodium lauryl sulphate or mixtures thereof.

According to these embodiments, the amount of the disintegrant in the compressed outer tablet layer is between 1.00% and 20.00%, preferably between 2.00 and 15.00%, more preferably between 2.00 and 10.00% by weight of the compressed outer tablet layer.

According to these embodiments, the amount of the disintegrant in the core tablet is between 1.00% and 20.00%, preferably between 2.00 and 15.00%, more preferably between 2.00 and 10.00% by weight of the core tablet.

The amounts of disintegrants, especially in the core tablet, are very important. In this present invention, the active agent dabigatran etexilate is in the outer layer and the core tablet is acidic. It is critical to ensure acidic environment. Therefore, disintegration of core tablet is a major parameter for bioavailability of the composition.

In one preferred embodiment, the disintegrant is selected from pregelatinized starch, croscarmellose sodium or mixtures thereof.

In one preferred embodiment, the disintegrant in the core tablet is pregelatinized starch.

In one preferred embodiment, the disintegrant in the core tablet is croscarmellose sodium.

Selection of pregelatinized starch or croscarmellose sodium as the disintegrant in the acidic core tablet also helps ensuring acidic environment in a short time.

In one preferred embodiment, the disintegrant in the compressed outer tablet layer is croscarmellose sodium.

Suitable lubricants are selected from a group comprising magnesium stearate, sodium stearyl fumarate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium lauryl sulphate or mixtures thereof.

In a preferred embodiment, the lubricant in the core tablet is magnesium stearate.

In a preferred embodiment, the lubricant in the compressed outer tablet layer is magnesium stearate.

According to these embodiments, the amount of lubricant in the compressed outer tablet layer is between 0.1% and 10.00%, preferably 0.1% and 5.00% and more preferably 0.5% and 2.00% by weight of the compressed outer tablet layer and the amount of the lubricant in the core tablet is between 0.1% and 10.00%, preferably 0.1% and 5.00% and more preferably 0.5% and 2.00% by weight of the core tablet.

Suitable glidants are selected from a group comprising colloidal silicon dioxide, talc, aluminium silicate or mixtures thereof.

In a preferred embodiment, the glidant in the core tablet is colloidal silicon dioxide.

In a preferred embodiment, the glidant in the compressed outer tablet layer is colloidal silicon dioxide.

According to these embodiments, the glidant is present in the compressed outer tablet layer in an amount of between 0.1% and 10.00%, preferably 0.1% and 5.00% and more preferably 0.1% and 2.00% by weight of the compressed outer layer and the glidant is present in the core tablet in an amount of between 0.1% and 10.00%, preferably 0.1% and 5.00% and more preferably 0.5% and 3.00% by weight of the core tablet.

Suitable fillers are selected from a group comprising microcrystalline cellulose, lactose, mannitol, spray-dried mannitol, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate, polyols, dextrose, maltitol or mixtures thereof.

In one preferred embodiment, the filler is microcrystalline cellulose in the core tablet and in the compressed outer tablet layer.

According to these embodiments, the amount of the filler in the compressed outer tablet layer is between 20.00 and 70.00%, preferably between 40.00% and 70.00%, more preferably between 50.00% and 65.00% by weight of the compressed outer tablet layer and the amount of the filler in the core tablet is between 20.00 and 70.00%, preferably between 40.00% and 70.00%, more preferably between 50.00% and 65.00% by weight of the core tablet.

Suitable binders are selected from the group comprising hydroxypropyl methyl cellulose, pregelatinized starch, povidone, copovidone, carnauba wax, pullulan, polymethacrylate, glyceryl behenate, hydroxypropyl cellulose, carboxymethyl cellulose, methyl cellulose, hydroxyethyl cellulose, sodium carboxymethyl cellulose, carboxymethyl cellulose calcium, ethyl cellulose, microcrystalline cellulose, polymetacrylates, polyethylene oxide, polyvinyl alcohol, polycarbophil, polyvinyl acetate and its copolymers, gelatin, starch, xanthan gum, guar gum, alginate, carrageen, collagen, agar, pectin, hyaluronic acid, carbomer, cellulose acetate phthalate, hydroxypropyl starch, hydroxyethyl methyl cellulose, poloxamer, polyethylene glycol, sugars, glycose syrups, natural gums, tragacanth gum, polyacrylamide, aluminum hydroxide, benthonite, laponite, cetostearyl alcohol, polyoxyethylene-alkyl ethers, acacia mucilage, polydextrose or mixtures thereof.

In one preferred embodiment, the binder in the compressed outer tablet layer is hydroxypropyl methylcellulose and the binder in the core tablet is hydroxypropyl methylcellulose.

According to these embodiments, the amount of the binder in the compressed outer tablet layer is between 0.50 and 20.00%, preferably 0.50 and 15.00%, more preferably 1.00 and 10.00% by weight of the compressed outer tablet layer.

According to these embodiments, the amount of the binder in the core tablet is between 0.10 and 5.00%, preferably 1.00 and 5.00% by weight of the core tablet.

According to one embodiment, wherein said one organic acid is selected from citric acid, tartaric acid, gallic acid, orotic acid, p-coumaric acid, hippuric acid, ferulic acid, vanillic acid, fumaric acid, maleic acid, succinic acid, malic acid, glutamic acid, aspartic acid, oxalic acid, lactic acid, formic acid, acetic acid, propionic acid, caproic acid, benzoic acid, carbonic acid or hydrates or salts or mixtures thereof.

According to this embodiment, preferably the organic acid is selected from citric acid and tartaric acid.

According to another embodiment, the organic acid is present in the core tablet in an amount of between 10.00% and 90.00, preferably between 15.00% and 70.00%, more preferably between 20.00% and 40.00% by weight of the core tablet.

According to one embodiment, the weight ratio of dabigatran etexilate free base or pharmaceutically acceptable salt of dabigatran etexilate to organic acid is between 0.5 and 4.0, preferably between 1.0 and 3.0, more preferably between 1.5 and 3.0.

Suitable salts of dabigatran etexilate are selected from a group comprising mesylate, maleate, malonate, citrate, tosylate, dabigatran esylate, tartrate, oxalate, camphor sulfonate.

Preferably suitable salts of dabigatran etexilate are selected from maleate, malonate, citrate, tosylate, esylate, tartrate, oxalate, camphor sulfonate.

In one embodiment, the pharmaceutical composition comprises;
a. dabigatran etexilate free base or a pharmaceutically acceptable salt of dabigatran etexilate 15.00-40.00%
b. croscarmellose sodium 2.00-10.00%
c. hydroxypropyl methylcellulose 1.00-10.00%
d. microcrystalline cellulose 50.00-65.00%
e. colloidal silicon dioxide 0.10-2.00%
f. magnesium stearate 0.50-2.00%
by weight of the compressed outer tablet layer
a. organic acid 20.00-40.00%
b. microcrystalline cellulose 50.00-65.00%
c. pregelatinized starch 2.00-10.00%
d. colloidal silicon dioxide 0.50-3.00%
e. magnesium stearate 0.50-2.00%
f. coating material 1.00-5.00%
by weight of the core.

In one embodiment, the pharmaceutical composition comprises;
a. dabigatran etexilate free base or a pharmaceutically acceptable salt of dabigatran etexilate 15.00-40.00%
b. croscarmellose sodium 2.00-10.00%
c. hydroxypropyl methylcellulose 1.00-10.00%
d. microcrystalline cellulose 50.00-65.00%
e. colloidal silicon dioxide 0.10-2.00%
f. magnesium stearate 0.50-2.00%
by weight of the compressed outer tablet layer
a. organic acid 20.00-40.00%
b. microcrystalline cellulose 50.00-65.00%
c. croscarmellose sodium 2.00-10.00%
d. colloidal silicon dioxide 0.50-3.00%
e. magnesium stearate 0.50-2.00%
f. coating material 1.00-5.00%
by weight of the core.

According to all these embodiments, the below given formulations can be used in the pharmaceutical composition subjected to the invention. These examples are not limiting the scope of the present invention and should be considered under the light of the foregoing detailed disclosure.

**Example 1: Tablet-in tablet**

| **Compressed outer tablet layer** | **(%) amount (w/w)** |
|---|---|
| Dabigatran etexilate free base or a pharmaceutically acceptable salt of dabigatran etexilate | 25.00 |
| Croscarmellose sodium | 5.00 |
| Hydroxypropyl methycellulose (5 cp) | 5.00 |
| Microcrystalline cellulose | 63.50 |
| Colloidal silicon dioxide | 0.50 |
| Magnesium stearate | 1.0 |
| **Total (compressed outer tablet layer)** | **100** |

| **Core tablet** | **(%) amount (w/w)** |
|---|---|
| Acid | 37.50 |
| Pregelatinized starch | 3.00 |
| Microcrystalline cellulose | 56.00 |
| Colloidal silicon dioxide | 2.50 |
| Magnesium stearate | 1.00 |
| **Total (core tablet)** | **100.0** |
| **Coating material** | **3.00** |
| Total (coating layer) | 100.00 |
| **Total (coated core tablet)** | **103.0** |
| | |

| **Coating material (Opadry Clear YS-1R-7006)** | **(%) amount (w/w)** |
|---|---|
| Hydroxypropyl methycellulose (5 cp) | 90.9 |
| Polyethylene glycol 400 | 4.55 |
| Polyethylene glycol 6000 | 4.55 |
| **Total coating material** | 100.00 |

The pharmaceutical compositions mentioned above are prepared by following these steps:
1. Preparation of the core tablets
   a. mixing pregelatinized starch, microcrystalline cellulose, colloidal silicon dioxide
   b. sieving and adding magnesium stearate to the mixture and mixing
   c. compressing the powder mixture into core tablets
   d. coating these core tablets
2. Preparation of the compressed outer tablet layer powder
   a. mixing dabigatran etexilate, croscarmellose sodium, hydroxypropyl methylcellulose, microcrystalline cellulose, colloidal silicon dioxide
   b. granulating with water
   c. drying and sieving
   d. adding magnesium stearate to the mixture and mixing
3. Preparation of the tablet-in-tablets
   a. filling one-half of the compressed outer tablet layer powder in a tablet-in-tablet compressing machine
   b. placing the core tablets on the compressed outer tablet layer powder in the compressing machine
   c. adding the other half of the compressed outer tablet layer powder on the core tablets in the compressing machine
   d. forming the final tablet-in-tablets by compressing
   e. coating these tablet-in-tablets

**Example 2: Tablet-in tablet**

| **Compressed outer tablet layer** | **(%) amount (w/w)** |
|---|---|
| Dabigatran etexilate free base or a pharmaceutically acceptable salt of dabigatran etexilate | 25.00 |
| Croscarmellose sodium | 5.00 |
| Hydroxypropyl methycellulose (5 cp) | 5.00 |
| Microcrystalline cellulose | 63.50 |
| Colloidal silicon dioxide | 0.50 |
| Magnesium stearate | 1.0 |
| **Total (compressed outer tablet layer)** | **100** |

| **Core tablet** | **(%) amount (w/w)** |
|---|---|
| Acid pellet | 30.00 |
| Microcrystalline cellulose | 60.00 |
| Croscarmellose sodium | 5.00 |
| Hydroxypropyl methycellulose (5 cp) | 3.00 |
| Colloidal silicon dioxide | 1.00 |
| Magnesium stearate | 1.00 |
| **Total (core tablet)** | **100.0** |
| **Total (coated core tablet)** | **103.0** |
| | |

| **Coating material (Opadry Clear YS-1R-7006)** | **(%) amount (w/w)** |
|---|---|
| Hydroxypropyl methycellulose (5 cp) | 90.9 |
| Polyethylene glycol 400 | 4.55 |
| Polyethylene glycol 6000 | 4.55 |
| **Total coating material** | 100.00 |

The pharmaceutical compositions mentioned above are prepared by following these steps:
1. Preparation of the core tablets
   a. coating the acid pellets
   b. mixing microcrystalline cellulose, croscarmellose sodium, hydroxypropyl methylcellulose, colloidal silicon dioxide and coated acid pellets
   c. adding magnesium stearate to the mixture and mixing
   d. compressing the mixture into the core tablets
2. Preparation of the compressed outer tablet layer powder
   a. mixing dabigatran etexilate, croscarmellose sodium, hydroxypropyl methylcellulose, microcrystalline cellulose, colloidal silicon dioxide
   b. granulating with water
   c. drying and sieving
   d. adding magnesium stearate to the mixture and mixing
3. Preparation of the tablet-in-tablets
   a. filling the half of the compressed outer tablet layer powder in a tablet-in-tablet compressing machine
   b. placing the core tablets on the compressed outer tablet layer powder in the compressing machine
   c. adding the other half of the compressed outer tablet layer powder on the core tablets in the compressing machine
   d. forming the final tablet-in-tablets by compressing
   e. coating these tablet-in-tablets

## Claims

1. A pharmaceutical composition comprising;
a. a core tablet comprising an organic acid and at least one pharmaceutically acceptable excipient
b. a compressed outer tablet layer covering the core tablet, comprising dabigatran etexilate free base or a pharmaceutically acceptable salt of dabigatran etexilate and at least one pharmaceutically acceptable excipient
wherein the core tablet has a coating and the weight ratio of the core tablet to the compressed outer tablet layer is between 0.1 and 0.5.

2. The pharmaceutical composition according to claim 1, wherein the pharmaceutical composition is in the form of a tablet-in-tablet.

3. The pharmaceutical composition according to claim 1, wherein said at least one pharmaceutically acceptable excipient is selected from a group comprising disintegrants, binders, fillers, lubricants, glidants or mixtures thereof.

4. The pharmaceutical composition according to claim 3, wherein the disintegrant is selected from a group comprising pregelatinized starch, cross-linked carboxymethyl cellulose, low-substituted hydroxypropyl cellulose , microcrystalline cellulose, sodium starch glycolate, starch, cross-linked polyvinyl N-pyrrolidone, poloxamer, sodium carboxymethyl cellulose, calcium carboxymethyl cellulose, carboxymethyl cellulose, docusate sodium, guar gum, polyacryline potassium, sodium alginate, alginic acid, alginates, ion-exchange resins, magnesium aluminium silica, sodium glycine carbonate, sodium lauryl sulphate or mixtures thereof.

5. The pharmaceutical composition according to claim 4, wherein the disintegrant in the core tablet is croscarmellose sodium.

6. The pharmaceutical composition according to claim 4, wherein the disintegrant in the core tablet is pregelatinized starch.

7. The pharmaceutical composition according to claim 4, wherein the disintegrant in the compressed outer tablet layer is croscarmellose sodium.

8. The pharmaceutical composition according to claim 1, wherein said organic acid is selected from citric acid, tartaric acid, gallic acid, orotic acid, p-coumaric acid, hippuric acid, ferulic acid, vanillic acid, fumaric acid, maleic acid, succinic acid, malic acid, glutamic acid, aspartic acid, oxalic acid, lactic acid, formic acid, acetic acid, propionic acid, caproic acid, benzoic acid, carbonic acid or hydrates or salts or mixtures thereof.

9. The pharmaceutical composition according to claim 1 or 8, wherein the weight ratio of dabigatran etexilate free base or pharmaceutically acceptable salt of dabigatran etexilate to the organic acid is between 0.5 and 4.0.

10. The pharmaceutical composition according to claim 8, wherein said organic acid is selected from citric acid and tartaric acid.

11. The pharmaceutical composition according to any of the preceding claims, comprising;
a. dabigatran etexilate free base or a pharmaceutically acceptable salt of dabigatran etexilate 15.00-40.00%
b. croscarmellose sodium 2.00-10.00%
c. hydroxypropyl methylcellulose 1.00-10.00%
d. microcrystalline cellulose 50.00-65.00%
e. colloidal silicon dioxide 0.10-2.00%
f. magnesium stearate 0.50-2.00%
by weight of the compressed outer tablet layer
a. organic acid 20.00-40.00%
b. microcrystalline cellulose 50.00-65.00%
c. pregelatinized starch 2.00-10.00%
d. colloidal silicon dioxide 0.50-3.00%
e. magnesium stearate 0.50-2.00%
f. coating material 1.00-5.00%
by weight of the core

12. A process for preparing the pharmaceutical composition according to claim 11, comprising the following steps:
a. Preparation of the core tablets
- mixing pregelatinized starch, microcrystalline cellulose, colloidal silicon dioxide
- sieving and adding magnesium stearate to the mixture and mixing
- compressing the powder mixture into core tablets
- coating these core tablets
b. Preparation of the compressed outer tablet layer powder
- mixing dabigatran etexilate, croscarmellose sodium, hydroxypropyl methylcellulose, microcrystalline cellulose, colloidal silicon dioxide
- granulating with water
- drying and sieving
- adding magnesium stearate to the mixture and mixing
c. Preparation of the tablet-in-tablets
- filling one-half of the compressed outer tablet layer powder in a tablet-in-tablet compressing machine
- placing the core tablets on the compressed outer tablet layer powder in the compressing machine
- adding the other half of the compressed outer tablet layer powder on the core tablets in the compressing machine
- forming the final tablet-in-tablets by compressing
- coating these tablet-in-tablets

13. The pharmaceutical composition according to claims 1 to 10, comprising;
a. dabigatran etexilate free base or a pharmaceutically acceptable salt of dabigatran etexilate 15.00-40.00%
b. croscarmellose sodium 2.00-10.00%
c. hydroxypropyl methylcellulose 1.00-10.00%
d. microcrystalline cellulose 50.00-65.00%
e. colloidal silicon dioxide 0.10-2.00%
f. magnesium stearate 0.50-2.00%
by weight of the compressed outer tablet layer
a. organic acid 20.00-40.00%
b. microcrystalline cellulose 50.00-65.00%
c. croscarmellose sodium 2.00-10.00%
d. colloidal silicon dioxide 0.50-3.00%
e. magnesium stearate 0.50-2.00%
f. coating material 1.00-5.00%
by weight of the core

14. A process for preparing the pharmaceutical composition according to claim 13 comprising the following steps:
a. Preparation of the core tablets
- coating the acid pellets
- mixing microcrystalline cellulose, croscarmellose sodium, hydroxypropyl methylcellulose, colloidal silicon dioxide and coated acid pellets
- adding magnesium stearate to the mixture and mixing
- compressing the mixture into the core tablets
b. Preparation of the compressed outer tablet layer powder
- mixing dabigatran etexilate, croscarmellose sodium, hydroxypropyl methylcellulose, microcrystalline cellulose, colloidal silicon dioxide
- granulating with water
- drying and sieving
- adding magnesium stearate to the mixture and mixing
c. Preparation of the tablet-in-tablets
- filling the half of the compressed outer tablet layer powder in a tablet-in-tablet compressing machine
- placing the core tablets on the compressed outer tablet layer powder in the compressing machine
- adding the other half of the compressed outer tablet layer powder on the core tablets in the compressing machine
- forming the final tablet-in-tablets by compressing
- coating these tablet-in-tablets

## Patentansprüche

1. Pharmazeutische Zusammensetzung, die aufweist:
a. eine Kerntablette, die eine organische Säure und mindestens einen pharmazeutisch akzeptablen Arzneistoffträger aufweist,
b. eine komprimierte äußere Tablettenschicht, die die Kerntablette bedeckt, die Dabigatranetexilat freien Grundstoff oder ein pharmazeutisch akzeptables Salz von Dabigatranetexilat und mindestens einen pharmazeutisch akzeptablen Arzneistoffträger aufweist,
wobei die Kerntablette einen Überzug hat und das Gewichtsverhältnis der Kerntablette zur komprimierten äußeren Tablettenschicht zwischen 0,1 und 0,5 liegt.

2. Pharmazeutische Zusammensetzung nach Anspruch 1,
wobei die pharmazeutische Zusammensetzung in Form einer Tablette-in-Tablette vorliegt.

3. Pharmazeutische Zusammensetzung nach Anspruch 1,
wobei der mindestens eine pharmazeutisch akzeptabler Arzneistoffträger aus einer Gruppe ausgewählt ist, die Zerfallsbeschleuniger (Sprengmittel), Bindemittel, Füllstoffe, Schmiermittel, Gleitmittel oder Mischungen davon aufweist.

4. Pharmazeutische Zusammensetzung nach Anspruch 3,
wobei der Zerfallsbeschleuniger (das Sprengmittel) aus einer Gruppe ausgewählt ist, die vorgelatinierte Stärke, vernetzte Carboxymethyl-Cellulose, niedrigsubstituierte Hydroxypropyl-Cellulose, mikrokristalline Cellulose, Natrium Stärke Glykolat, Stärke, vernetztes Polyvinyl N-Pyrrolidon, Poloxamer, Natrium Carboxymethyl-Cellulose, Calcium-Carboxymethyl-Cellulose, Carboxymethyl-Cellulose, Docusat-Natrium, Guargummi, Polyacrylin-Kalium, Natrium-Alginat, Alginsäure, Alginate, lonenaustausch-Harze, Magnesium-Aluminium-Kieselsäure, Natrium-Glycin Carbonat, Natrium-Lauryl-Sulfat oder Mischungen davon aufweist.

5. Pharmazeutische Zusammensetzung nach Anspruch 4,
wobei der Zerfallsbeschleuniger (das Sprengmittel) in der Kerntablette Croscarmellose-Natrium ist.

6. Pharmazeutische Zusammensetzung nach Anspruch 4,
wobei das Sprengmittel in der Kerntablette vorgelatinierte Stärke ist.

7. Pharmazeutische Zusammensetzung nach Anspruch 4,
wobei der Zerfallsbeschleuniger (das Sprengmittel) in der komprimierten äußeren Tablettenschicht Croscarmellose-Natrium ist.

8. Pharmazeutische Zusammensetzung nach Anspruch 1,
wobei die organische Säure ausgewählt ist aus Zitronensäure, Weinsäure, Gallussäure, Orotsäure, p-Cumarsäure, Hippursäure, Ferulasäure, Vanillinsäure, Fumarsäure, Maleinsäure, Bernsteinsäure, Äpfelsäure, Glutaminsäure, Asparaginsäure, Oxalsäure, Milchsäure, Ameisensäure, Essigsäure, Propionsäure, Capronsäure, Benzoesäure, Kohlensäure oder Hydrate oder Salze oder Mischungen davon.

9. Pharmazeutische Zusammensetzung nach Anspruch 1 oder 8,
wobei das Gewichtsverhältnis von Dabigatranetexilat freie Grundstoff oder pharmazeutisch akzeptables Salz von Dabigatranetexilat zu der organischen Säure zwischen 0,5 und 4,0 liegt.

10. Pharmazeutische Zusammensetzung nach Anspruch 8,
wobei die organische Säure ausgewählt ist aus Zitronensäure und Weinsäure.

11. Pharmazeutische Zusammensetzung nach einem der vorhergehenden Ansprüche, die aufweist:
a. den Dabigatranetexilat freien Grundstoff oder ein pharmazeutisch akzeptables Salz von Dabigatranetexilat mit 15,00-40,00%;
b. Croscarmellose-Natrium mit 2,00-10,00%
c. Hydroxypropylmethyl-Cellulose mit 1,00-10,00%
d. mikrokristalline Zellulose mit 50,00-65,00%.
e. kolloidales Siliciumdioxid mit 0,10-2,00%.
f. Magnesiumstearat mit 0,50-2,00%.
bezogen auf das Gewicht der gepressten äußeren Tablettenschicht;
a. organische Säure mit 20,00-40,00%
b. mikrokristalline Cellulose mit 50,00-65,00%
c. vorgelatinierte Stärke mit 2,00-10,00%
d. kolloidales Siliciumdioxid mit 0,50-3,00%.
e. Magnesiumstearat mit 0,50-2,00%.
f. Beschichtungsmaterial mit 1,00-5,00%
bezogen auf das Gewicht des Kerns.

12. Verfahren zur Herstellung der pharmazeutischen Zusammensetzung nach Anspruch 11, das folgenden Schritte aufweist:
a. Zubereitung der Kerntabletten:
- Mischen von vorgelatinierter Stärke, mikrokristalliner Cellulose, kolloidalem Siliciumdioxid;
- Sieben und Zugeben von Magnesiumstearat zu der Mischung und Mischen;
- Verpressen der Pulvermischung zu Kerntabletten;
- Beschichten dieser Kerntabletten;
b. Zubereitung des Pulvers der komprimierten äußeren Tablettenschicht:
- Vermischen von Dabigatranetexilat, Croscarmellose-Natrium, Hydroxypropylmethyl-Cellulose, mikrokristalliner Cellulose, kolloidalem Siliciumdioxid;
- Granulieren mit Wasser;
- Trocknen und Sieben;
- Zugeben von Magnesiumstearat zu der Mischung und Mischen;
c. Zubereitung der Tabletten-in-Tabletten:
- Einfüllen einer Hälfte des gepressten äußeren Tablettenschichtpulvers in eine Tabletten-in-Tablette Pressmaschine;
- Auflegen der Kerntabletten auf das komprimierte äußere Tablettenschichtpulver in der Pressmaschine;
- Zufügen der anderen Hälfte des komprimierten äußeren Tablettenschichtpulvers auf die Kerntabletten in der Pressmaschine;
- Formen der fertigen Tablette-in-Tabletten durch Verpressen;
- Beschichten dieser Tablette-in-Tabletten.

13. Pharmazeutische Zusammensetzung nach einem der Ansprüche 1 bis 10, die aufweist:
a. Dabigatranetexilat freier Grundstoff oder ein pharmazeutisch akzeptables Salz von Dabigatranetexilat mit 15,00-40,00%;
b. Croscarmellose-Natrium mit 2,00-10,00%;
c. Hydroxypropylmethyl-Cellulose mit 1,00-10,00%;
d. mikrokristalline Zellulose mit 50,00-65,00%;
e. kolloidales Siliciumdioxid mit 0,10-2,00%;
f. Magnesiumstearat mit 0,50-2,00%;
bezogen auf das Gewicht der gepressten äußeren Tablettenschicht;
a. organische Säure mit 20,00-40,00%;
b. mikrokristalline Cellulose mit 50,00-65,00%;
c. Croscarmellose-Natrium mit 2,00-10,00%;
d. kolloidales Siliciumdioxid mit 0,50-3,00%;
e. Magnesiumstearat mit 0,50-2,00%;
f. Beschichtungsmaterial mit 1,00-5,00%;
bezogen auf das Gewicht des Kerns.

14. Verfahren zur Herstellung der pharmazeutischen Zusammensetzung nach Anspruch 13, das die folgenden Schritte aufweist:
a. Zubereitung der Kerntabletten;
- Beschichten der Säurepellets;
- Mischen von mikrokristalliner Cellulose, Croscarmellose-Natrium, Hydroxypropylmethyl-Cellulose, kolloidales Siliciumdioxid und beschichtete Säurepellets;
- Zugeben von Magnesiumstearat zu der Mischung und Mischen;
- Verpressen der Mischung zu Kerntabletten;
b. Zubereitung des Pulvers für die komprimierte äußere Tablettenschicht:
- Vermischen von Dabigatranetexilat, Croscarmellose-Natrium, Hydroxypropylmethyl-Cellulose, mikrokristalliner Cellulose, kolloidalem Siliciumdioxid;
- Granulieren mit Wasser;
- Trocknen und Sieben;
- Zugeben von Magnesiumstearat zu der Mischung und Mischen;
c. Zubereitung der Tablette-in-Tabletten:
- Einfüllen der Hälfte des gepressten äußeren Tablettenschichtpulvers in eine Tablette-in-Tablette Pressmaschine;
- Auflegen der Kerntabletten auf das komprimierte äußere Tablettenschichtpulver in der Pressmaschine;
- Zufügen der anderen Hälfte des gepressten äußeren Tablettenschichtpulvers auf die Kerntabletten in der Pressmaschine;
- Formen der fertigen Tablette-in-Tabletten durch Verpressen;
- Beschichten dieser Tablette-in-Tabletten;

## Revendications

1. - Composition pharmaceutique comprenant ;
a. un comprimé de noyau comprenant un acide organique et au moins un excipient pharmaceutiquement acceptable ;
b. une couche de comprimé externe comprimée recouvrant le comprimé de noyau, comprenant du dabigatran étexilate base libre ou un sel pharmaceutiquement acceptable de dabigatran étexilate et au moins un excipient pharmaceutiquement acceptable,
dans laquelle le comprimé de noyau a un enrobage et le rapport en poids du noyau de comprimé à la couche de comprimé externe comprimée est entre 0,1 et 0,5.

2. - Composition pharmaceutique selon la revendication 1, dans laquelle la composition pharmaceutique se présente sous la forme d'un comprimé dans un comprimé.

3. - Composition pharmaceutique selon la revendication 1, dans laquelle ledit au moins un excipient pharmaceutiquement acceptable est choisi dans un groupe comprenant les délitants, les liants, les charges, les lubrifiants, les agents de glissement ou les mélanges de ceux-ci.

4. - Composition pharmaceutique selon la revendication 3, dans laquelle le délitant est choisi dans un groupe comprenant l'amidon prégélatinisé, la carboxyméthyl cellulose réticulée, l'hydroxypropyl cellulose faiblement substituée, la cellulose microcristalline, le glycolate d'amidon sodique, l'amidon, la polyvinyl N-pyrrolidone réticulée, un poloxamère, la carboxyméthyl cellulose sodique, la carboxyméthyl cellulose calcique, la carboxyméthyl cellulose, le docusate de sodium, la gomme de guar, le polyacrylate de potassium, l'alginate de sodium, l'acide alginique, les alginates, les résines échangeuses d'ions, le silicate de magnésium et d'aluminium, le carbonate de glycine sodique, le lauryl sulfate de sodium ou les mélanges de ceux-ci.

5. - Composition pharmaceutique selon la revendication 4, dans laquelle le délitant dans le comprimé de noyau est la croscarmellose sodique.

6. - Composition pharmaceutique selon la revendication 4, dans laquelle le délitant dans le comprimé de noyau est l'amidon prégélatinisé.

7. - Composition pharmaceutique selon la revendication 4, dans laquelle le délitant dans la couche de comprimé externe comprimée est la croscarmellose sodique.

8. - Composition pharmaceutique selon la revendication 1, dans laquelle ledit acide organique est choisi parmi l'acide citrique, l'acide tartrique, l'acide gallique, l'acide orotique, l'acide p-coumarique, l'acide hippurique, l'acide férulique, l'acide vanillique, l'acide fumarique, l'acide maléique, l'acide succinique, l'acide malique, l'acide glutamique, l'acide aspartique, l'acide oxalique, l'acide lactique, l'acide formique, l'acide acétique, l'acide propionique, l'acide caproïque, l'acide benzoïque, l'acide carbonique ou les hydrates ou les sels ou les mélanges de ceux-ci.

9. - Composition pharmaceutique selon l'une des revendications 1 ou 8, dans laquelle le rapport en poids du dabigatran étexilate base libre ou du sel pharmaceutiquement acceptable de dabigatran étexilate à l'acide organique est entre 0,5 et 4,0.

10. - Composition pharmaceutique selon la revendication 8, dans laquelle ledit acide organique est choisi parmi l'acide citrique et l'acide tartrique.

11. - Composition pharmaceutique selon l'une quelconque des revendications précédentes, comprenant ;
a. du dabigatran étexilate base libre ou un sel pharmaceutiquement acceptable de dabigatran étexilate 15,00 à 40,00 %
b. de la croscarmellose sodique 2,00 à 10,00 %
c. de l'hydroxypropyl méthylcellulose 1,00 à 10,00 %
d. de la cellulose microcristalline 50,00 à 65,00 %
e. du dioxyde de silicium colloïdal 0,10 à 2,00 %
f. du stéarate de magnésium 0,50 à 2,00 %
en poids de la couche de comprimé externe comprimée
a. de l'acide organique 20,00 à 40,00 %
b. de la cellulose microcristalline 50,00 à 65,00%
c. de l'amidon prégélatinisé 2,00 à 10,00 %
d. du dioxyde de silicium colloïdal 0,50 à 3,00 %
e. du stéarate de magnésium 0,50 à 2,00 %
f. un matériau d'enrobage 1,00 à 5,00%
en poids du noyau.

12. - Procédé de préparation de la composition pharmaceutique selon la revendication 11, comprenant les étapes suivantes :
a. Préparation des comprimés de noyau ;
- mélange d'amidon prégélatinisé, de cellulose microcristalline, de dioxyde de silicium colloïdal ;
- tamisage et addition de stéarate de magnésium au mélange et mélangeage ;
- compression du mélange de poudres en comprimés de noyau ;
- enrobage de ces comprimés de noyau ;
b. Préparation de la poudre de couche de comprimé externe comprimée :
- mélange de dabigatran étexilate, de croscarmellose sodique, d'hydroxypropyl méthylcellulose, de cellulose microcristalline, de dioxyde de silicium colloïdal ;
- granulation avec de l'eau ;
- séchage et tamisage ;
- addition de stéarate de magnésium au mélange et mélangeage ;
c. Préparation des comprimés - dans - comprimés :
- remplissage par la moitié de la poudre de couche de comprimé externe comprimée d'une machine de compression de comprimés - dans - comprimés ;
- mise en place des comprimés de noyau sur la poudre de couche de comprimé externe comprimée dans la machine de compression ;
- addition de l'autre moitié de la poudre de couche de comprimé externe comprimée sur les comprimés de noyau dans la machine de compression ;
- formage des comprimés - dans - comprimés finaux par compression ;
- enrobage de ces comprimés - dans - comprimés.

13. - Composition pharmaceutique selon l'une des revendications 1 à 10, comprenant ;
a. du dabigatran étexilate base libre ou un sel pharmaceutiquement acceptable de dabigatran étexilate 15,00 à 40,00 %
b. de la croscarmellose sodique 2,00 à 10,00 %
c. de l'hydroxypropyl méthylcellulose 1,00 à 10,00 %
d. de la cellulose microcristalline 50,00 à 65,00 %
e. du dioxyde de silicium colloïdal 0,10 à 2,00 %
f. du stéarate de magnésium 0,50 à 2,00 %
en poids de la couche de comprimé externe comprimée
a. de l'acide organique 20,00 à 40,00 %
b. de la cellulose microcristalline 50,00 à 65,00 %
c. de la croscarmellose sodique 2,00 à 10,00 %
d. du dioxyde de silicium colloïdal 0,50 à 3,00 %
e. du stéarate de magnésium 0,50 à 2,00 %
f. un matériau d'enrobage 1,00 à 5,00 %
en poids du noyau.

14. - Procédé de préparation de la composition pharmaceutique selon la revendication 13, comprenant les étapes suivantes :
a. Préparation des comprimés de noyau :
- enrobage des pastilles d'acide ;
- mélange de cellulose microcristalline, de croscarmellose sodique, d'hydroxypropyl méthylcellulose, de dioxyde de silicium colloïdal et des pastilles d'acide enrobées ;
- addition de stéarate de magnésium au mélange et mélangeage ;
- compression du mélange en comprimés de noyau ;
b. Préparation de la poudre de couche de comprimé externe comprimée :
- mélange de dabigatran étexilate, de croscarmellose sodique, d'hydroxypropyl méthylcellulose, de cellulose microcristalline, de dioxyde de silicium colloïdal ;
- granulation avec de l'eau ;
- séchage et tamisage ;
- addition de stéarate de magnésium au mélange et mélangeage ;
c. Préparation des comprimés - dans - comprimés :
- remplissage par la moitié de la poudre de couche de comprimé externe comprimée d'une machine de compression de comprimés - dans - comprimés ;
- mise en place des comprimés de noyau sur la poudre de couche de comprimé externe comprimée dans la machine de compression ;
- addition de l'autre moitié de la poudre de couche de comprimé externe comprimée sur les comprimés de noyau dans la machine de compression ;
- formage des comprimés - dans - comprimés finaux par compression ;
- enrobage de ces comprimés - dans - comprimés.
